(19)

Europäisches
Patentamt
European
Patent Office
Office européen
des brevets

(11) **EP 2 279 409 B1**

(12) **EUROPEAN PATENT SPECIFICATION**

(45) Date of publication and mention
of the grant of the patent:
**04.04.2012 Bulletin 2012/14**

(51) Int Cl.:
***G01N 33/28*** *(2006.01)*

(21) Application number: **09751466.5**

(22) Date of filing: **20.05.2009**

(86) International application number:
**PCT/US2009/044668**

(87) International publication number:
**WO 2009/143238 (26.11.2009 Gazette 2009/48)**

(54) **A PROCESS FOR DETERMINING THE DISTILLATION CHARACTERISTICS OF A LIQUID PETROLEUM PRODUCT CONTAINING AN AZEOTROPIC MIXTURE**

VERFAHREN ZUR BESTIMMUNG DER DESTILLATIONSEIGENSCHAFTEN EINES FLÜSSIGEN ERDÖLPRODUKTS MIT EINER AZEOTROPEN MISCHUNG

PROCÉDÉ DE DÉTERMINATION DES CARACTÉRISTIQUES DE DISTILLATION D'UN PRODUIT PÉTROLIER LIQUIDE CONTENANT UN MÉLANGE AZÉOTROPE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **22.05.2008 US 55284**

(43) Date of publication of application:
**02.02.2011 Bulletin 2011/05**

(73) Proprietor: **BUTAMAX ADVANCED BIOFUELS LLC**
**Wilmington DE 19803 (US)**

(72) Inventor: **WOLF, Leslie, Raymond**
**Warrenville**
**IL 60555 (US)**

(74) Representative: **Towler, Philip Dean**
**Dehns**
**St Bride's House**
**10 Salisbury Square**
**London**
**EC4Y 8JD (GB)**

(56) References cited:
• **GLINDEMANN D ET AL: "Role of azeotropy in characterization of complex hydrocarbon mixtures by true-boiling-point distillation" FLUID PHASE EQUILIBRIA 1997 AUG ELSEVIER SCI B.V., vol. 135, no. 2, August 1997 (1997-08), pages 149-167, XP7910037 ISSN: 0378-3812**

• **ROEKENS EDWARD J L ET AL: "AZEOTROPIC DATA FOR THE SYSTEMS FORMED BY REPRESENTATIVES OF THE HOMOLOGOUS SERIES OF ALKANES AND ALCOHOLS" J APPL CHEM BIOTECHNOL 1976 NOV, vol. 26, no. 11, November 1976 (1976-11), pages 595-610, XP7910039**

• **LEE LIANG-SUN ET AL: "Prediction of azeotrope by activity coefficient models without parameters" JOURNAL OF THE CHINESE INSTITUTE OF CHEMICAL ENGINEERS 1996 JUL CHINESE INST OF CHEMICAL ENGINEERS, vol. 27, no. 4, July 1996 (1996-07), pages 295-315, XP8113060 ISSN: 0368-1653**

• **SMITH B L ET AL: "Improvements in the measurement of distillation curves. 3. Application to gasoline and gasoline + methanol mixtures" INDUSTRIAL AND ENGINEERING CHEMISTRY RESEARCH 20070103 AMERICAN CHEMICAL SOCIETY US, vol. 46, no. 1, 3 January 2007 (2007-01-03), pages 297-309, XP7910033 ISSN: 0888-5885**

• **DE MENEZES E W ET AL: "Addition of an azeotropic ETBE/ethanol mixture in eurosuper-type gasolines" FUEL DECEMBER 2006 ELSEVIER LTD GB, vol. 85, no. 17-18, December 2006 (2006-12), pages 2567-2577, XP25235872 ISSN: 0016-2361**

**Description**

<u>Background of the Invention</u>

This invention relates to a process for producing a liquid petroleum product that contains an azeotropic mixture of an oxygenated or nitrogen containing component and at least one petroleum blending component.

Distillation and Reid vapor pressure properties of gasoline and diesel fuel oil influence their performance such as cold start, warm-up, and deposit forming tendency, and their emissions such as evaporative and engine-out exhaust. In fact these volatility characteristics are subjects of regulation by both the United States Environmental Protection Agency and the states that require ASTM D4814 gasoline standard and/or ASTM D975 diesel standard. In order to produce gasoline and diesel fuel oil with the most economic blend in view of a refinery's operating constraints refiners make use of models to predict the properties of the resulting final blends based upon the properties of the available blending stocks. Therefore a model that accurately predicts the properties of a blend is an important tool. In particular, models for predicting distillation characteristics are important because the gasoline distillation points T10, T50 and T90 and the diesel fuel oil distillation point T90 have specifications depending on the season and geographic location. Glindemann et al, Fluid Phase Equilibria, 1997, vol. 135, pages 149-167, discloses a method for describing quantatively the azeotropic effect, presuming distillation of pure compounds or of azeotropic mixtures.

For conventional gasoline and diesel fuel, the blending model can be relatively straightforward because the hydrocarbon blending stocks behave nearly ideally, and their mixture's vapor pressure follows Raoults' Law or does so with minor modifications. However, the vapor pressure of oxygenated fuels, particularly alcohols such as ethanol, propanol and butanols, and esters, ketones, ethers, ester alcohols, keto-alcohols, ether alcohols, aldehydes, ether aldehydes, aldehyde alcohols, etc., are non-ideal, and therefore the blend models for such oxygenated fuels are not simple. Nitrogen containing compounds such as amines, amides, nitriles, nitro esters, etc. are also known to form non-ideal mixtures with hydrocarbons and therefore their blend models are not simple. It would be highly desirable to develop a blending model for accurately predicting the distillation characteristics for petroleum products that contain azeotropic mixtures.

<u>Summary of the Invention</u>

The present invention is a process for producing a liquid petroleum product that contains an azeotropic mixture of an oxygenated or nitrogen containing component and at least one petroleum blending component, comprising: (I) determining the distillation characteristics of the liquid petroleum product by: (a) determining the mathematical relationship between the boiling points of hydrocarbons between specified minimum and maximum hydrocarbon boiling temperatures and the concentration of each such hydrocarbon in its binary azeotrope with the oxygenated or nitrogen containing component (b) determining the mathematical relationship between the boiling points of the aforesaid hydrocarbons and the boiling points of such binary azeotropes between the aforesaid minimum and maximum hydrocarbon boiling temperatures; (c) dividing the boiling point curve of the combined at least one petroleum blending component between the aforesaid minimum and maximum hydrocarbon boiling points into narrow volume percent distillate fractions to thereby provide a defined distillation temperature for each such volume percent distillate fraction; (d) for each aforesaid volume percent distillate fraction from step (c), (i) from the relationship from step (a), determining the total concentration of hydrocarbons in the distillate fraction; (ii) from the total concentration of hydrocarbons from step (d)(i) and starting from the lowest aforesaid volume percent distillate fraction, determining the amounts of the aforesaid azeotropic mixture and of the oxygenated or nitrogen containing component in the distillate fraction for each such volume percent distillate fraction; and (iii) from the relationship from step (b), determining the boiling point of the aforesaid azeotropic mixture that corresponds to each such volume percent distillate fraction; and (e) for each aforesaid volume percent distillate fraction correlating the amount of the azeotropic mixture in the distillate fraction from step (d)(ii) with the boiling point from step (d)(iii), and combining such correlations to thereby determine the distillation characteristics of the aforesaid liquid petroleum product; and (II) blending the azeotropic mixture and at least one petroleum blending component to produce the liquid petroleum product having distillation characteristics determined in step (e).

<u>Brief Description of the Drawings</u>

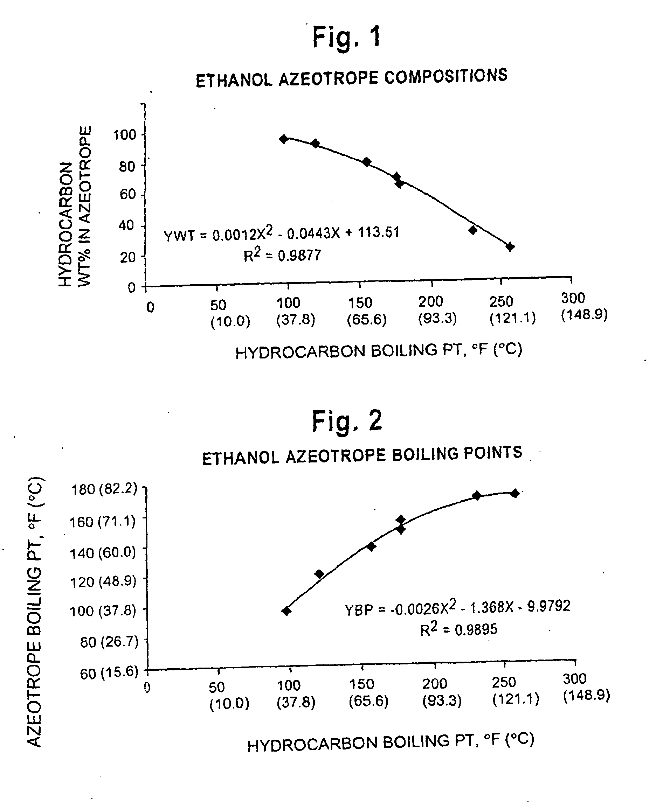

<u>Figure 1</u> is a plot of the concentration of each of the hydrocarbons in Table 1 in its azeotrope with ethanol versus the boiling point of each such hydrocarbon.

<u>Figure 2</u> is a plot of the boiling point of the azeotrope with ethanol of each hydrocarbon in Table 1 versus the boiling point of each such hydrocarbon.

Figure 3 is a plot of the observed distillation characteristics and calculated distillation characteristics for a blend containing gasoline and 10 volume percent of fuels grade ethanol.

Figure 4 is a plot of the observed distillation characteristics and calculated distillation characteristics for a blend containing gasoline and 20 volume percent of fuels grade ethanol.

Figure 5 is a plot of the observed distillation characteristics and calculated distillation characteristics for a blend containing gasoline and 40 volume percent of fuels grade ethanol.

Figure 6 is a plot of the observed distillation characteristics and calculated distillation characteristics for a blend containing gasoline and 60 volume percent of fuels grade ethanol.

Detailed Description of the Preferred Embodiments

Gasolines and diesel fuel oils are well known in the art and generally contain as a primary component a mixture of hydrocarbons having different boiling points and typically boiling under atmospheric pressure at temperatures in the range of from about 79 °F (26°C) to about 437 °F (225°C) for gasolines and in the range of from about 360°F (182°C) to about 710°F (377°C) for diesel fuel oils. These ranges are such

approximate and can vary depending upon the actual mixture of hydrocarbon molecules present, the additives or other compounds present (if any), and the environmental conditions. Oxygenated gasolines and oxygenated diesel fuel oils are blends of either a gasoline blend stock or a diesel fuel oil blend stock and one or more oxygenates.

While gasoline and diesel fuel containing relatively high levels, for example, greater than 1%, of nitrogen compounds are not common, bio-derived blending components containing nitrogen are being produced, such as nitriles derived from vegetable oils described by Western Biofuels, Inc. as "high energy biodiesel" (HEBD), http://www.westernbiofuelsinc.com/index.html. Future bio-derived blending components could contain significant amounts of nitrogen because of their biological origin. A blend model for these compounds would be advantageous to evaluate their impact on blend distillation characteristics.

Gasoline and diesel fuel blend stocks can be produced from a single component, such as the product from a refinery alkylation unit or other refinery streams. However, gasoline and diesel fuel blend stocks are more commonly blended using more than one component. Gasoline and diesel fuel blend stocks are blended to meet desired physical and performance characteristics and to meet regulatory requirements and may involve a few components, for example three or four, or may involve many components, for example twelve or more. Gasolines, diesel fuels, and gasoline and diesel fuel blend stocks optionally may include other chemicals or additives. For example, additives or other chemicals can be added to adjust properties of a gasoline or diesel fuel to meet regulatory requirements, add or enhance desirable properties, reduce undesirable detrimental effects, adjust performance characteristics, or otherwise modify the characteristics of the gasoline or diesel fuel. Examples of such chemicals or additives include detergents, antioxidants, stability enhancers, demulsifiers, corrosion inhibitors, metal deactivators, lubricity improvers, friction modifiers, cold flow improvers and others. More than one additive or chemical can be used. Useful additives and chemicals are described in Colucci et al., U.S. Patent No. 5,782,937. Such additives and chemicals are also described in Wolf, U.S. Patent No. 6,083,228, Ishida et al., U.S. Patent No. 5,755,833, U.S. Patent No. 5,858028, U.S. Patent No. 5,997,592, 6,248,142, U.S. Patent No. 6,280,488 and U.S. Patent No. 6,277,159. Gasolines, diesel fuels and gasoline or diesel fuel blend stocks may also contain solvent or carrier solutions which are often used to deliver additives into a fuel.

Gasoline and diesel fuel blend stocks suitable for use in the method of this invention are typically blend stocks useable for making gasolines and diesel fuels for consumption in spark or compression ignition engines or in other engines which combust gasoline or diesel fuel. Suitable gasoline blend stocks include blend stocks for gasolines meeting ASTM 4814 and blend stocks for reformulated gasoline. Suitable gasoline blend stocks also include blend stocks having low a sulfur content which may be desired to meet regional requirements, for example, having less than about 150, preferably less than about 100, and more preferably less than about 80 parts per million parts by volume of sulfur. Such suitable gasoline blend stocks also include blend stocks having low aromatics content which may be desirable to meet regulatory requirements, for example, having less than about 8000 and preferably less than about 7000 parts per million parts by volume of benzene, or for example, having less than about 35 and more preferably less than about 25 volume percent total of all aromatic species. Suitable diesel fuel blend stocks include blend stocks for diesel fuels meeting ASTM D975. Suitable diesel fuel blend stocks include light middle distillate or kerosene, heavy middle distillate, light catalytic cracker cycle oil, coke still distillate, light and heavy hydrocracker distillates, and hydrotreater distillates. Also, such diesel fuel oil blend stocks may be blended together as feed to a hydrosulfurization unit to reduce sulfur level as required by regulations. The product stream from such a hydrodesulfurization unit can then be used as a suitable diesel fuel oil component to blend with oxygenate .

An oxygenate such as ethanol can also be blended with the gasoline or diesel fuel blending stock at any point within the distribution chain. For example, the one or more blending stocks and one or more suitable oxygenates can be combined in a refinery, or the one or more suitable blending stocks can be combined in a refinery and then transported to a terminal where the one or more suitable oxygenates can be blended with the gasoline or diesel fuel blend stock.

For the purpose of illustration only, the method of this invention will be exemplified with gasoline and with an oxygenated component, in particular ethanol. The first two steps of the method of the present invention involve determining two mathematical relationships between specified minimum and maximum boiling temperatures. The specific minimum and maximum boiling temperatures depend on the identity of the specific oxygenated component involved. The specified minimum and maximum temperatures for ethanol are 95°F (35°C) and 280°F (138°C), respectively. The first is the mathematical relationship between the boiling points of the hydrocarbons boiling between the specified minimum and maximum hydrocarbon boiling temperatures with the concentration of each such hydrocarbon in its binary azeotrope with an aforesaid alcohol component, preferably ethanol or one or more isomers of propanol or butanol or mixtures thereof, and more preferably ethanol. The second is the mathematical relationship between the boiling points of the aforesaid hydrocarbons and the boiling points of such binary azeotropes. Such hydrocarbons that form azeotropes with ethanol are n-pentane, cyclopentane, n-hexane, cyclohexane, benzene, toluene, and n-octane. The boiling points of such hydrocarbons, the boiling points of their binary azeotropes with ethanol, both at atmospheric pressure, and their concentrations in such azeotropes are presented in Table 1.

Table 1

| Hydrocarbons | Hydrocarbon Boiling Point (°F) | (°C) | Binary Azeotrope | | |
| --- | --- | --- | --- | --- | --- |
| | | | Boiling Point (°F) | (°C) | Hydrocarbon Concentration (Wt%) |
| n-pentane | 96.98 | 36.1 | 97.07 | 36.2 | 95 |
| cyclopentane | 120.56 | 49.2 | 129.92 | 54.4 | 92.5 |
| n-hexane | 156.02 | 68.9 | 137.62 | 58.7 | 79 |
| cyclohexane | 177.26 | 80.7 | 148.64 | 64.8 | 64 |
| benzene | 176.18 | 80.1 | 154.22 | 67.9 | 68.3 |
| toluene | 231.08 | 110.6 | 170.06 | 76.7 | 32 |
| n-octane | 258.26 | 125.7 | 170.6 | 77.0 | 22 |

The data in Table 1 is used to make the plots shown in Figures 1 and 2. The plot in Figure 1 is represented by the following Equation 1, where x is the hydrocarbon boiling point (°F) and ywt is the hydrocarbon concentrate in its binary azeotrope.

$$ywt = -0.0012x^2 - 0.0443x + 113.51 \qquad \text{Equation 1}$$

with $R^2$ for Equation 1 being equal to 0.9877. The total concentration of hydrocarbons in the azeotrope can be determined from the boiling point of its hydrocarbon components using Equation 1. The plot in Figure 2 is represented by the following Equation 2 where x is the hydrocarbon boiling point (°F) and ybp is the boiling point (°F) of its binary azeotrope.

$$ybp = -0.0026x^2 + 1.368x - 9.979 \quad \text{Equation 2}$$

with $R^2$ for Equation 2 being equal to 0.9895. The boiling point of the azeotrope can be determined from the boiling point of its hydrocarbon components using Equation 2.

Next the boiling point curve, for example, as measured by the ASTM D86 method, of the combined petroleum blending component of the liquid petroleum product is divided between the minimum and maximum temperatures specified in step a) into narrow volume percent distillate fractions to thereby provide a defined distillation temperature for each such volume percent distillate fraction. Thus, if several petroleum blending components are to be combined to make the liquid petroleum product, it is the boiling point curve of the combined blending components that is divided in this step. By contrast, if the liquid petroleum product contains only one petroleum blending component, the boiling point curve of that single blending component is divided in this step.

The boiling curve may be divided by various methods. A basic procedure is to use the temperature volume percent distilled data from the ASTM D86 method by assigning 5 volume percent to the initial boiling point (IBP) temperature; then 10 volume percent to the 10 percent distilled temperature; then 10 volume percent to the 20 percent distilled temperature; and repeating this assignment procedure until the 90 percent distillation temperature is assigned and finally assigning 5 volume percent to the final boiling point (FBP) temperature. Thus, the so divided curve is defined by eleven narrow boiling fractions, nine of 10 volume percent each and two of 5 volume percent each, and their corresponding boiling points. This basic procedure can be modified by making smaller volume percent assignments and assigning appropriate additional temperatures intermediate to the usual ASTM D86 data by interpolation of the boiling curve. Other modifications of the basic procedure include using alternate boiling curve data such as true boiling point (TBP) ASTM D285 or simulated distillation ASTM D2892. Also, there are well known procedures to convert one type of data to the other, for example converting ASTM D86 data to TBP data. Modifications of the basic procedure can be used individually or in combinations to improve agreement of the predictions and/or facilitate speed of calculation.

Thereafter in the fourth step of the method of this invention, for each of the aforesaid narrow volume percent distillate fractions in the boiling point curve so divided in the third step:

In a first substep of this fourth step, the total concentration of hydrocarbons in the narrow volume percent distillate fraction is determined using the mathematical relationship determined in the aforesaid first step. Equation 1 is the relationship for ethanol as shown in Table 1.

In a second substep, from this total concentration of hydrocarbons and starting from the lowest narrow volume percent distillate fraction, the amounts of the aforesaid azeotropic mixture and of the alcohol in the distillate fraction are determined by straightforward calculation. An adjustable parameter may be employed in this substep to account for any hydrocarbons that do not form azeotropes with the oxygenate, as evidenced by differences between the observed boiling point curve and the boiling point curve calculated in the fifth step described below. No such adjustment was necessary in this example.

In a third substep, the boiling point of the aforesaid azeotropic mixture that corresponds to such narrow volume percent distillate fraction is determined using the mathematical relationship determined in the aforesaid step 2. Equation 2 is the relationship for ethanol as shown in Table 1.

In the fifth step, the amount of the azeotropic mixture in the distillate fraction from the aforesaid second substep of the fourth step is correlated with the boiling point from the aforesaid third substep of the fourth step for each aforesaid narrow volume percent distillate fraction, and such correlations are combined to thereby determine the distillation characteristics of the aforesaid liquid petroleum product. The proportion of oxygenate to the hydrocarbon blend component limits the amount of azeotropes that are formed by conservation of mass. Excess oxygenate, if any, is treated as an additional pure blending component (that is, at its normal boiling point). Thus, the method of the present invention permits the distillation characteristics of complex hydrocarbon mixtures of unknown compositions to be predicted accurately from data for known hydrocarbons.

Distillations illustrating the method of this invention were performed using the blend stock whose distillation (ASTM D86) is shown in Table 2 with various amounts of fuel grade ethanol, which contained 95 volume percent of ethanol and 5 volume percent of a hydrocarbon denaturant.

Table 2
Hydrocarbon Blending Component

| Volume % Distilled | Temperature, | |
|---|---|---|
| | °F | °C |
| Initial Boiling Point | 95.9 | 35.5 |
| 10 | 123.3 | 50.7 |
| 20 | 134.6 | 57.0 |
| 30 | 147.8 | 64.3 |
| 40 | 163.2 | 72.9 |
| 50 | 184.9 | 84.9 |
| 60 | 214.7 | 101.5 |
| 70 | 260.7 | 127.1 |
| 80 | 322.7 | 161.5 |
| 90 | 348.8 | 176.0 |
| Final Boiling Point | 412 | 211.1 |

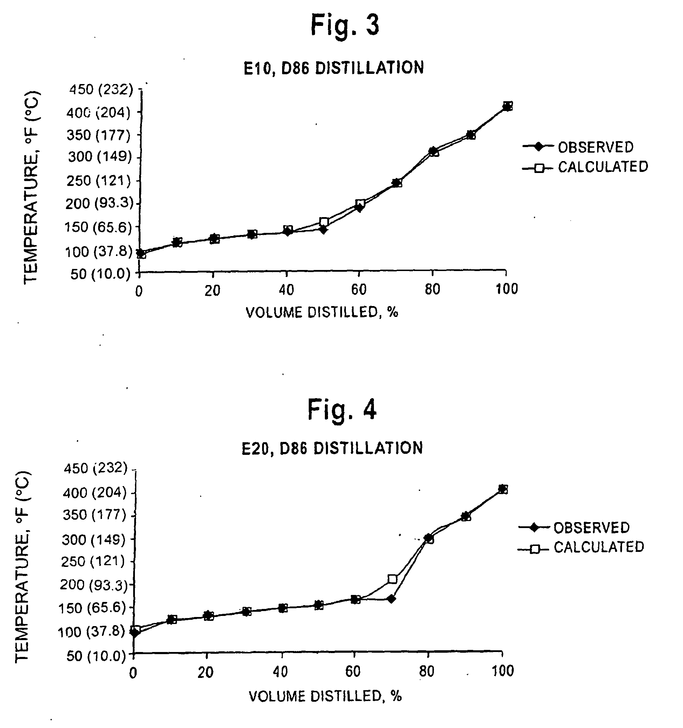

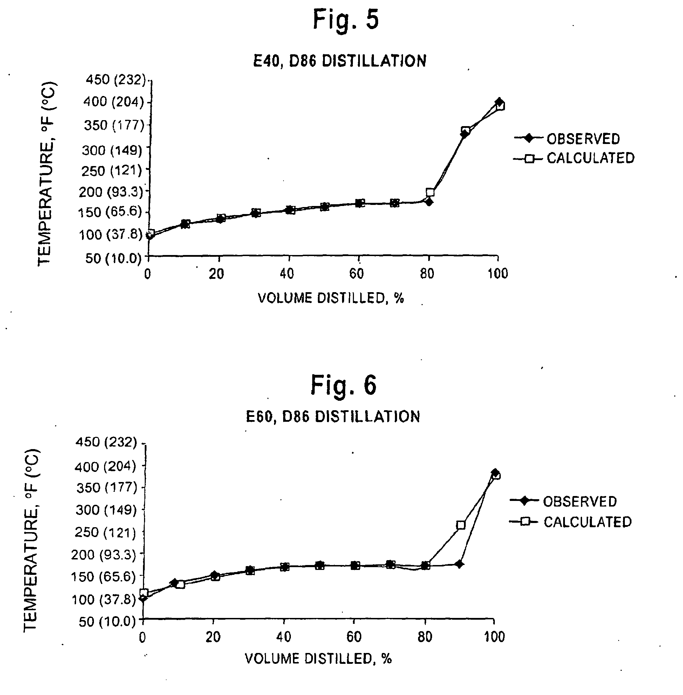

Figures 3 through 6 show comparisons between the observed distillations and the distillation characteristics calculated using the method of this invention. Figures 3, 4, 5, and 6 illustrate distillations of the aforesaid blends containing 10, 20, 40 and 60 volume percent, respectively (E10, E20, E40, E60), of the fuel grade ethanol. Figures 3 - 6 demonstrate excellent agreement between the observed distillation results and the calculated distillation characteristics. For the blends containing the lower concentrations of 10 and 20 volume percent of ethanol, the calculated temperatures match the observed azeotrope boiling temperatures, while for the blends containing the higher concentrations of 40 and 60 volume percent of ethanol demonstrate the ethanol boiling at both its azeotrope temperatures and its normal boiling point (173°F) (78°C) because no more hydrocarbon is available to form azeotropes. The largest deviations between the observed and calculated boiling temperatures are at the steep change in the curve where experimental variability is the highest.

In these examples, the hydrocarbon blending component distillation curve was characterized by its ASTM D86 data. As stated hereinabove, it is well known to those skilled in the art that other distillation characterizations, such as "true boiling point" (TBP, like ASTM D285) and simulated distillation (ASTM D2892) can be used to advantage when calculating distillation properties of a final mixture from the distillation properties of its constituent components. In addition, the well known mathematical method of splines can be used to obtain smooth curves for either the division of a curve into narrow components or the combination of a collection of narrow components into a composite curve.

It will be appreciated by those skilled in the art that, while the present invention has been described herein by reference to particular methods, materials, and specific examples, the scope of the present invention is limited only by the claims.

**Claims**

1. A process for producing a liquid petroleum product that contains an azeotropic mixture of an oxygenated or nitrogen containing component and at least one petroleum blending component, the process comprising:

   (I) determining the distillation characteristics of the liquid petroleum product by:

   (a) determining the mathematical relationship between the boiling points of hydrocarbons between specified minimum and maximum hydrocarbon boiling temperatures and the concentration of each such hydrocarbon in its binary azeotrope with the oxygenated or nitrogen containing component;
   (b) determining the mathematical relationship between the boiling points of the aforesaid hydrocarbons and the boiling points of such binary azeotropes between the aforesaid minimum and maximum boiling temperatures;
   (c) dividing the boiling curve of the combined at least one petroleum blending component between the aforesaid minimum and maximum temperatures into narrow volume percent distillate fractions to thereby provide a defined distillation temperature for each such volume percent distillate fraction;
   (d) for each aforesaid volume percent distillate fraction from step (c)

   (i) from the relationship from step (a), determining the total concentration of hydrocarbons in the distillate fraction;
   (ii) from the total concentration of hydrocarbons from step (d)(i) and starting from the lowest boiling point aforesaid volume percent distillate fraction, determining the amounts of the aforesaid azeotropic mixture and of the oxygenated or nitrogen containing component in each such volume percent distillate fraction and
   (iii) from the relationship from step (b), determining the boiling point of the aforesaid azeotropic mixture that corresponds to each such volume percent; and

   (e) correlating for each aforesaid volume percent distillate fraction the amount of the azeotropic mixture in the distillate fraction from step (d)(ii) with the boiling point from step (d)(iii), and combining such correlations to thereby determine the distillation characteristics of the aforesaid liquid petroleum product; and

   (II) blending the azeotropic mixture and the at least one petroleum blending component to produce the liquid petroleum product having the distillation characteristics determined in step (e).

2. The process of Claim 1 wherein the oxygenated component is an alcohol, ester, ketone, ether, ester alcohol, keto-alcohol, ether alcohol, aldehyde, ether aldehyde, or aldehyde alcohol.

3. The process of Claim 2 wherein the oxygenated component is at least one alcohol.

4. The process of Claim 3 wherein the oxygenated component is ethanol.

5. The process of Claim 4 wherein the aforesaid oxygenate is ethanol and specified minimum and maximum hydrocarbon component boiling temperatures are 95°F and 280°F, respectively.

6. The method of Claim 3 wherein the oxygenated component comprises at least one of ethanol, an isomer of propanol or an isomer of butanol.

7. The process of Claim 1 wherein the liquid petroleum product is a gasoline.

8. The process of Claim 2 wherein the oxygenated component is a mixture of one or more oxygenates.

9. The process of Claim 8 wherein the oxygenated component is a mixture of one or more alcohols.

10. The process of Claim 1 wherein the nitrogen containing component is an amine, amide, nitrile, nitro ester, nitrate ester, nitrite ester, cyclic nitrogen compound, amino alcohol, ether amine, or poly amine.

11. The process of Claim 10 wherein the nitrogen containing component is a mixture of one or more nitrogen containing components.

12. The process of Claim 1 wherein the nitrogen containing component is a mixture of one or more nitrogen containing components and one or more oxygenated components.

**Patentansprüche**

1. Verfahren zum Herstellen eines flüssigen Erdölprodukts, das eine azeotrope Mischung einer mit Sauerstoff angereicherten oder stickstoffhaltigen Komponente und mindestens einer Erdölmischkomponente enthält, wobei das Verfahren Folgendes umfasst:

   (I) das Bestimmen der Destillationseigenschaften des flüssigen Erdölprodukts durch

   (a) Bestimmen des mathematischen Zusammenhangs zwischen den Siedepunkten von Kohlenwasserstoffen zwischen spezifierten Mindest- und Höchstkohlenwasserstoff-Siedetemperaturen und der Konzentration jedes derartigen Kohlenwasserstoffs in seinem binären Azeotrop mit der mit Sauerstoff angereicherten oder stickstoffhaltigen Komponente;
   (b) Bestimmen des mathematischen Zusammenhangs zwischen den Siedepunkten der obigen Kohlenwasserstoffe und den Siedepunkten derartiger binärer Azeotrope zwischen den oben erwähnten Mindest- und Höchstsiedetemperaturen;
   (c) Teilen der Siedekurve der kombinierten mindestens einen Erdölmischkomponente zwischen den oben erwähnten Mindest- und Höchsttemperaturen in enge Volumenprozent-Destillatfraktionen, um dadurch eine definierte Destillationstemperatur für jede derartige Volumenprozent-Destillationsfraktion bereitzustellen;
   (d) für jede der oben erwähnten Volumenprozent-Destillatfrakionen aus Schritt (c)

   (i) Bestimmen, aus dem Zusammenhang aus Schritt (a), der Gesamtkonzentration von Kohlenwasserstoffen in der destillierten Fraktion;
   (ii) Bestimmen, aus der Gesamtkonzentration von Kohlenwasserstoffen aus Schritt (d)(i) und ausgehend von der oben erwähnten Destillatfraktion mit dem niedersten Siedepunkt, der Mengen der oben erwähnten azeotropen Mischung und der mit Sauerstoff angereicherten oder stickstoffhaltigen Komponente in jeder derartigen Volumenprozent-Destillatfraktion und
   (iii) Bestimmen, aus dem Zusammenhang aus Schritt (b), des Siedepunkts der oben erwähnten azeotropen Mischung, die jedem derartigen Volumenprozentsatz entspricht; und

   (e) Korrelieren, für jede obenerwähnte Volumenprozent-Destillatfraktion, der Menge der azeotropen Mischung in der Destillatfraktion aus Schritt (d)(ii) mit dem Siedepunkt aus Schritt (d)(iii) und Kombinieren derartiger Korrelationen, um dadurch die Destillationseigenschaften des oben erwähnten flüssigen Erdölprodukts zu bestimmen; und

(II) das Mischen der azeotropen Mischung und der mindestens einen Erdölmischkomponente um das flüssige Erdölprodukt herzustellen, das die in Schritt (e) bestimmten Destillationseigenschaften aufweist.

2. Verfahren nach Anspruch 1, wobei die mit Sauerstoff angereicherte Komponente ein Alkohol, Ester, Keton, Ether, Esteralkohol, Ketoalkohol, Etheralkohol, Aldehyd, Etheraldehyd oder Aldehydalkohol ist.

3. Verfahren nach Anspruch 2, wobei die mit Sauerstoff angereicherte Komponente mindestens ein Alkohol ist.

4. Verfahren nach Anspruch 3, wobei die mit Sauerstoff angereicherte Komponente Ethanol ist.

5. Verfahren nach Anspruch 4, wobei das oben erwähnte Oxygenat Ethanol ist und die spezifizierten Mindest- und Höchstsiedetemperaturen der Kohlenwasserstoffkomponente 95 °C bzw. 280 °F betragen.

6. Verfahren nach Anspruch 3, wobei die mit Sauerstoff angereicherte Komponente mindesten eines von Ethanol, einem Isomer von Propanol oder einem Isomer von Butanol umfasst.

7. Verfahren nach Anspruch 1, wobei das flüssige Erdölprodukt Gasolin ist.

8. Verfahren nach Anspruch 2, wobei die mit Sauerstoff angereicherte Komponente eine Mischung von einem oder mehreren Oxygenaten ist.

9. Verfahren nach Anspruch 8, wobei die mit Sauerstoff angereicherte Komponente eine Mischung von einem oder mehreren Alkoholen ist.

10. Verfahren nach Anspruch 1, wobei die stickstoffhaltige Komponente ein Amin, Amid, Nitril, Nitroester, Nitratester, Nitritester, eine cyclische Stickstoffkomponente, Aminoalkohol, Etheramid oder Polyamin ist.

11. Verfahren nach Anspruch 10, wobei die stickstoffhaltige Komponente eine Mischung von einer oder mehreren stickstoffhaltigen Komponenten ist.

12. Verfahren nach Anspruch 1, wobei die stickstoffhaltige Komponente eine Mischung von einer oder mehreren stickstoffhaltigen Komponenten und einer oder mehreren mit Sauerstoff angereicherten Komponenten ist.

**Revendications**

1. Procédé de production d'un produit pétrolier liquide qui contient un mélange azéotrope d'un composant oxygéné ou contenant de l'azote et au moins un composant de mélange pétrolier, le procédé comprenant:

(I) la détermination des caractéristiques de distillation du produit pétrolier liquide par:

(a) la détermination de la relation mathématique existant entre les points d'ébullition des hydrocarbures entre les températures d'ébullition minimale et maximale spécifiées de l'hydrocarbure et la concentration de chacun de ces tels hydrocarbures en son azéotrope binaire avec le composant oxygéné ou contenant de l'azote;
(b) la détermination de la relation mathématique entre les points d'ébullition des hydrocarbures susmentionnés et les points d'ébullition de tels azéotropes binaires entre les températures d'ébullition minimale et maximale précédemment mentionnées;
(c) la division de la courbe d'ébullition du au moins un composant de mélange pétrolier combiné entre les températures minimale et maximale précédemment mentionnées en fractions étroites de distillat en pourcentage en volume pour fournir de là une température définie de distillation pour chacune de telle fraction de distillat en pourcentage en volume;
(d) pour chaque fraction précédemment mentionnée de distillat en pourcentage en volume provenant de l'étape (c)

(i) de la relation de l'étape (a), la détermination de la concentration totale des hydrocarbures dans la fraction de distillat;
(ii) à partir de la concentration totale des hydrocarbures de l'étape (d)(i) et en commençant à partir de la fraction précédemment mentionnée de distillat en pourcentage en volume à point d'ébullition le plus bas, la détermination des quantités du mélange azéotrope précédemment mentionné et du composant oxygéné ou contenant de l'azote dans chacune de telle fraction de distillat en pourcentage en volume et (iii) à partir de la relation de l'étape (b), la détermination du point d'ébullition du mélange azéotrope précédemment mentionné qui correspond à chacun de tel pourcentage en volume; et

(e) la corrélation pour chaque fraction précédemment mentionnée de distillat en pourcentage en volume de la quantité du mélange azéotrope dans la fraction de distillat à partir de l'étape (d)(ii) au point d'ébullition à partir de l'étape (d)(iii), et la combinaison de telles corrélations pour déterminer de là les caractéristiques de distillation du produit pétrolier liquide précédemment mentionné; et

(II) le mélange du mélange azéotrope et d'au moins un composant de mélange pétrolier pour produire le produit pétrolier liquide ayant les caractéristiques de distillation déterminées dans l'étape (e).

2. Procédé selon la revendication 1, dans lequel le composant oxygéné est un alcool, un ester, une cétone, un éther, un ester-alcool, un céto-alcool, un éther-alcool, un aldéhyde, un éther-aldéhyde ou un aldéhyde-alcool.

3. Procédé selon la revendication 2, dans lequel le composant oxygéné est au moins un alcool.

4. Procédé selon la revendication 3, dans lequel le composant oxygéné est l'éthanol.

5. Procédé selon la revendication 4, dans lequel l'oxygénat précédemment mentionné est l'éthanol et les températures d'ébullition minimale et maximale spécifiées du composant hydrocarbure sont respectivement de 95°F et 280°F.

6. Procédé selon la revendication 3, dans lequel le composant oxygéné comprend au moins l'un parmi l'éthanol, un isomère de propanol ou un isomère de butanol.

7. Procédé selon la revendication 1, dans lequel le produit pétrolier liquide est une essence.

8. Procédé selon la revendication 2, dans lequel le composant oxygéné est un mélange d'un ou plusieurs oxygénat(s).

9. Procédé selon la revendication 8, dans lequel le composant oxygéné est un mélange d'un ou plusieurs alcool(s).

10. Procédé selon la revendication 1, dans lequel le composant contenant de l'azote est une amine, un amide, un nitrile, un nitro-ester, un nitrate-ester, un nitrite-ester, un composé cyclique azoté, un alcool aminé, une éther-amine ou une poly-amine.

11. Procédé selon la revendication 10, dans lequel le composant contenant de l'azote est un mélange d'un ou plusieurs composant(s) contenant de l'azote.

12. Procédé selon la revendication 1, dans lequel le composant contenant de l'azote est un mélange d'un ou plusieurs composant(s) contenant de l'azote et un ou plusieurs composant(s) oxygénés.

# Fig. 1

### ETHANOL AZEOTROPE COMPOSITIONS

$YWT = 0.0012X^2 - 0.0443X + 113.51$

$R^2 = 0.9877$

HYDROCARBON WT% IN AZEOTROPE

HYDROCARBON BOILING PT, °F (°C)

# Fig. 2

### ETHANOL AZEOTROPE BOILING POINTS

$YBP = -0.0026X^2 - 1.368X - 9.9792$

$R^2 = 0.9895$

AZEOTROPE BOILING PT, °F (°C)

HYDROCARBON BOILING PT, °F (°C)

## Fig. 3

### E10, D86 DISTILLATION

## Fig. 4

### E20, D86 DISTILLATION

# Fig. 5

**E40, D86 DISTILLATION**

# Fig. 6

**E60, D86 DISTILLATION**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5782937 A, Colucci
- US 6083228 A, Wolf
- US 5755833 A, Ishida
- US 5858028 A
- US 5997592 A
- US 6248142 B
- US 6280488 B
- US 6277159 B

**Non-patent literature cited in the description**

- **GLINDEMANN et al.** *Fluid Phase Equilibria,* 1997, vol. 135, 149-167